# EUROPEAN PATENT APPLICATION

(11) **EP 1 970 365 A1**
(43) Date of publication of application: **17.09.2008**
(21) Application number: 07104217.0
(22) Date of filing: 15.03.2007
(51) Int. Cl.: C07C 45/79, C07C 49/17, A61K 8/35, A61Q 17/04

(54) **Method for stabilizing liquid dihydroxyacetone**

(71) Applicant: PURAC Biochem BV, 4206 AC Gorinchem (NL)
(72) Inventor: Kremer, Diderik Reinder, 9722 WG, GRONINGEN (NL); Vorage, Marcus Johannes Anthonius Wilhelmus, 9458 TC, BALLOO (NL)
(74) Representative: Beetz, Tom

(57) **Abstract**

The invention pertains to a method for stabilizing liquid dihydroxyacetone (DHA) by subjecting crude liquid DHA to an ion exchange process on a cation exchange resin, followed by subjecting the liquid DHA to an anion exchange resin. By using a weak anion exchange resin, which is pretreated with an acid the stabilized liquid DHA is obtained with high purity. The invention further relates to the stabile liquid DHA thus obtained, syrup containing the same, and the use thereof for the manufacture of a cosmetic product.

## Description

The invention pertains to a method for purifying and stabilizing liquid dihydroxyacetone, to pure and stabile liquid dihydroxyacetone, to syrup containing the same and to the use of said syrup for the manufacture of cosmetic products.

It is known for a long time, for instance from US 2,948,658 and references cited therein, that dihydroxyacetone (DHA) solutions can be produced by fermentative oxidation of glycerol. The stability of such DHA solutions is a problem. Upon storage of such solutions the DHA content decreases and by-products are formed. Furthermore, DHA is obtained by fermentative oxidation of glycerol as a colored product and therefore should be decolorized, for instance by treatment with active carbon.

A method for obtaining decolorized DHA was disclosed in US 4,076,589, wherein the crude DHA was subjected to a cation exchange and an anion exchange process. According to this procedure isolation of DHA was performed with cation exchange resin Amberlite IRC-120, followed by anion exchange resin Amberlite IRA-401S. It is stressed that this method was only used to decolorize crude DHA to colorless DHA, which was immediately crystallized to obtain a stable crystalline product.

It is therefore an objective of the invention to provide a method for obtaining decolorized liquid DHA and at the same time such method should provide stabile liquid DHA for which crystallization is unnecessary. It was now found that the method of US 4,076,589, apart for its use to decolorize crude DHA, could also be used to obtain a stable liquid product. However, it was also found that products, which are obtained colorless by this method and which remain colorless when shelved for a few months, are not sufficiently stable as a liquid over longer periods under normal storage conditions, such as a year or more at room temperature. Upon such longer shelve periods the liquid DHA was found to decompose to colored and colorless decomposition products. Thus it was now found that a method wherein crude liquid DHA was subjected to a cation exchange process, followed by an anion exchange process could also be used for stabilizing liquid dihydroxyacetone (DHA). Since by using this method DHA was obtained without substantial color, but decomposition to colorless and colored products occurred after storage for longer periods (such as for instance, during a year or more at room temperature, it was also an objective to improve the above method to obtain a process wherein the stability was further improved to allow shelving of liquid DHA for a year or more, without decomposition to colored and colorless decomposition products. It was further found that the known method is suitable to decolorize crude DHA, but that the application thereof leads to the formation of new colorless impurities.

It is therefore a further objective to prevent formation of uncolored byproducts, which objective was met by using the previously mentioned method; however by using an anion exchange resin which is pretreated with an acid prior to its use in the stabilization method. Preferably, the anion exchange resin is a weak anion exchange resin, which is pretreated with an acid.

It was found that the use of an acid-pretreated weak anion exchange resin leads to DHA of higher purity than previously obtained by the method of US 4,076,598, as could be monitored by the presence or absence of a shoulder in the DHA peak of an HPLC chromatogram. The preferred method according to the invention wherein the anion exchange resin is pretreated with acid, therefore does not only lead to improved color stability and stability in general, but further provides DHA of higher purity than when using an anion exchange resin which was not pretreated by an acid. The term high-purity means that the purity of DHA is at least 97%, more preferably at least 98%, and most preferably at least 99%, as determined by HPLC.

Pretreatment can be done with any suitable acid, including inorganic acids such as diluted sulfuric acid and diluted phosphoric acid, but organic acids such as acetic acid, lactic acid, citric acid, gluconic acid, and the like, are preferred. Lactic acid is particularly suitable.

Pretreatment of the resin can be done in a fixed bed, or by expanding the bed to a vessel on top of the column. Well mixing of the expanded bed could be achieved by introducing compressed air into the bottom of the column. Pretreatment was followed by pH measurement, to obtain the optimum pH value between 3 and 4. Therefore dosing lactic acid best could be done in small portions for a number of hours.

Suitable anion exchange resins are weak anion exchange resins. Weak anion exchange resins are known in the art. Ion-exchange columns are categorized as either strong or weak. Strong ion exchange columns are charged (ionized) at all pH levels while weak ion exchange columns are only ionized in a certain pH range. The term "weak anion exchanger" relates to exchange resins that are moderately basic, usually having either a tertiary or secondary amine functional group, such as DEAE (diethylaminoethyl), and not having quaternary ammonium groups as are present in strong anion exchange resins. Examples of weak anion exchange resins are Imac A24, IMAC HP661, Amberlite FPA 51, Amberlite FPA 53, Amberlite FPA 55, Purolite A-100S, Purolite A-103S, Purolite A-123S, and weak anion exchange resins available under the trade names Amberlyst, Dowex, Duolite, Permutit, and Chempro.

The cation purification step is preferably performed using a strong cation exchange resin. Strong cation exchange resins are known in the art. The term "strong cation exchanger" relates to exchange resins that have a strong acidic functional group, such as sulfonium ions. Examples of strong cation exchange resins are Imac C8P, Amberlite 120 H, Amberlite FPC 22H, and strong cation exchange resins available under the trade names Amberlyst, Dowex, Duolite, Permutit, and Chempro.

Without pretreatment of the anion exchange resin byproducts may be formed during the chromatography process. It is believed that his occurs according to known processes which have been disclosed by P.W. Kent and K.R. Wood (1976), Trihydric alcohols, their analogues and derivatives and their oxidation products: trihydric alcohols to triketones (Rod's Chemistry of Carbon Compounds. Vol. 1, Part E, Chapter 18). Thus dependent on the pH DHA can be converted to byproducts like methylglyoxal and glyceraldehyde.

We have now found that the acid pretreatment of the anion exchange resin leads to high-purity products that do not contain substantial amounts of byproducts (less than 3% or better). These solutions further can be stored for long periods without occurrence of colorization and formation of colorless decomposition products. These stability and purity conditions allow commercial exploitation of these solutions. Thus in another aspect the invention also pertains to the use of DHA solutions as obtained according to the method of this invention for instance for use in cosmetic products. This is a considerable advantage over the existing practice wherein DHA is first crystallized to obtain a color-stable product, which crystals later in the process of manufacturing cosmetic products are dissolved again to a suitable solution. The storage and use of ready-to-use solutions, usually concentrated viscous syrupy solutions, provide a substantial advantage when making cosmetic products.

It is also an object of the invention to provide a stabile liquid DHA obtainable by the method of the invention wherein the increase of the color value (CV) as determined spectrophotometrically is less than 50%, preferably less than 10%, more preferably less than 5% after 42 days of storage at 25 °C. Most preferably such solutions are syrups that can easily contain 60 to 70% (w/v) DHA.

The invention is further illustrated by the following non-limitative examples.

An ion exchange chromatography installation was build as a manually operating system. The filtered liquid was cooled to 4 °C and filtered with a polishing filter before entering the cation exchange column. The liquid from the cation exchanger was directly transferred to the anion exchange column. The discharge of the anion exchanger was stored. To avoid pressure build-up the ion exchanger was open at the top. The whole flow through the cation and anion exchanger was based on gravity.

After filtration of a DHA-containing fermentation liquid, the solution was passed through cation and anion exchange columns and the discharge was used for color stability measurements. As reference fermentation filtrate was measured that had not passed through the ion exchange system. Both liquids were concentrated by evaporation at temperatures below 40 °C to about 60% DHA (w/v).

### Table 1:

### Color stability of ion exchange treated and untreated liquid DHA (60% w/v)

### APHA color of ion exchange treated liquid DHA:

| | Time (days) | | | | | | |
|---|---|---|---|---|---|---|---|
| | 0 | 3 | 6 | 14 | 21 | 32 | 42 |
| Stored at 6 °C | 18 | 18 | 18 | 17 | 17 | 17 | 16 |
| Stored at 25 °C | 18 | 18 | 18 | 18 | 18 | 18 | 19 |

### APHA color of ion exchange untreated liquid DHA:

| | Time (days) | | | | | | |
|---|---|---|---|---|---|---|---|
| | 0 | 3 | 6 | 14 | 21 | 32 | 42 |
| Stored at 6 °C | 86 | 87 | 86 | 86 | 84 | 88 | 86 |
| Stored at 25 °C | 86 | 178 | 270 | 476 | 706 | 942 | 1060 |

APHA (American Public Health Agency) color was determined according to NEN-EN-ISO 7887; 1994 en, which is identical to e.g. ASTM D 1209 (PT-Co scale).

After pretreatment of the anion exchange material with lactic acid, as described above, cation treated filtered fermentation liquid was passed through the anion exchange column and the discharge was used for impurity profile determination. As reference, material that had passed through a non-pretreated anion exchange column was measured. The results are given in Table 2:

| | % DHA | |
|---|---|---|
| | at t=0 | after 42 days at 25 °C |
| No ion exchange | 99 | 89 |
| Ion exchange without pretreatment | 99 | 94 |
| Ion exchange according to the invention | 99 | 99* |
| | | 96** |

| | | |
|---|---|---|
| * 32 days ** 90 days | | |

## Claims

1. A method for stabilizing liquid dihydroxyacetone (DHA) by subjecting crude liquid DHA to an ion exchange process on a cation exchange resin, followed by subjecting the liquid DHA to an anion exchange resin.

2. The method according to claim 1 for obtaining stabilized liquid high-purity DHA wherein the anion exchange resin is a weak anion exchange resin which is pretreated with an acid.

3. The method according to claim 2 wherein the acid is lactic acid.

4. The method according to claim 2 or 3 wherein the resin is pretreated at a pH between 3 and 4.

5. The method according to claim 1 wherein crude liquid DHA is stabilized by performing the steps:
(a) treating a weak anion exchange resin with an acid;
(b) ion exchanging the crude liquid DHA over a cation exchange resin; followed by
(c) ion exchanging the liquid DHA over the anion exchange resin as obtained in step (a).

6. The method according to claim 5 wherein step (a) is performed with lactic acid at pH 3-4.

7. A stabile liquid DHA obtainable by the method of claim 1 wherein the increase of the color value (CV) determined spectrophotometrically is less than 50%, preferably less than 10%, most preferably less than 5% after 32 days of storage at 25 °C.

8. Syrup comprising the stabile liquid DHA as obtained by the method of claim 1.

9. Use of the stabile liquid DHA as obtained by the method of claim 1 or of the syrup of claim 8 for the manufacture of a cosmetic product.
